# FASCICULE DE BREVET EUROPEEN

(11) **EP 1 156 761 B1**
(45) Date de publication et mention de la délivrance du brevet: **23.06.2004**
(21) Numéro de dépôt: 00906439.5
(22) Date de dépôt: 21.02.2000
(51) Int. Cl.: A61F 2/16

(54) **DISPOSITIF POUR L'INJECTION D'UNE LENTILLE INTRA-OCULAIRE EN MATIERE SOUPLE**
IMPLANTATIONSWERKZEUG ZUM IMPLANTIEREN EINER FLEXIBLEN INTRAOKULARLINSE
DEVICE FOR INJECTING AN INTRAOCULAR LENS MADE OF FLEXIBLE MATERIAL

(30) Priorité: 22.02.1999 FR 9902602
(43) Date de publication de la demande: 28.11.2001
(73) Titulaire: Laboratoire de Contactologie Appliquée - LCA, 28000 Chartres (FR)
(72) Inventeur: VINCENT, Patrice, F-28130 Mevoisins (FR)
(74) Mandataire: CAPRI
(86) Numéro de dépôt international: PCT/FR2000/000425
(87) Numéro de publication internationale: WO 2000/049974

(56) Documents cités:
- WO-A-96/13229
- US-A- 4 787 904
- US-A- 4 955 889
- US-A- 5 275 604

## Description

La présente invention concerne un dispositif pour injecter, après extraction du cristallin,' une lentille intra-oculaire en matière souple préalablement déformée par compression, roulage ou pliage.

La plupart des injecteurs de lentilles intra-oculaires connus jusqu'à présent comportent un corps cylindrique dans lequel un piston est monté coulissant ou vissant : ce corps est conçu pour recevoir une cartouche comportant un embout cylindrique, une chambre de chargement pour la lentille à injecter et une ailette articulée ; on place la lentille dans la chambre et on rabat l'ailette pour fermer la chambre, ce qui déforme la lentille, puis on place la cartouche dans le corps ; l'embout étant engagé par le chirurgien dans une incision de l'oeil du patient, l'action sur le piston permet d'injecter la lentille directement dans le sac capsulaire de l'oeil opéré. Une fois libérée, la lentille reprend sa forme initiale.

On connaît également d'autres injecteurs comportant une chambre de chargement munie d'une ouverture d'accès pouvant être obturée par un volet, un tiroir ou par le montage de son embout La déformation de la lentille est obtenue soit en refermant le volet ou le tiroir, soit par la poussée directe du piston.

Dans tous les cas, le piston propulse la lentille dans un espace dont la section se réduit progressivement, ce qui contribue à déformer la lentille, jusqu'à atteindre la section interne minimale de l'embout.

Le document WO 96/13229 divulgue un dispositif comportant deux parties, à savoir une pince et un élément tubulaire chacune devant être tenue d'une main. L'utilisateur saisit une lentille avec la pince et l'introduit dans une chambre de chargement de l'élément tubulaire.

La présente invention a pour objet un injecteur qui ne comporte pas de chambre ou système de chargement avec accès direct (tel que cartouche, volet, tiroir, embout amovible ...), et dans lequel la déformation de la lentille fait appel à la seule poussée directe du piston.

L'injecteur selon l'invention est caractérisé par un corps de seringue monobloc comportant une première partie cylindrique de section approximativement semi-circulaire pouvant contenir une lentille non-déformée, un embout d'injection et une partie intermédiaire raccordant ces deux parties et dont la section diminue progressivement depuis la premiére partie cylindrique jusqu'à l'embout. La section de l'embout, qui peut être circulaire, ovoïde ou aplatie, a ses dimensions adaptées à la largeur des incisions pratiquées dans la technique chirurgicale de la phacoémulsification (couramment 3,2 mm voire moins selon l'évolution des techniques).

Dans un mode de réalisation préférée de l'invention, l'extrémité d'éjection du piston comporte plusieurs brins pouvant par flexibilité se rapprocher les uns des autres, lors du déplacement du piston, et pousser simultanément la lentille dans l'embout. Grâce à cette disposition, la poussée sur la lentille s'exerce en plusieurs points, ce qui stabilise son orientation. Le piston est réalisé en une seule pièce, en matière plastique dure, la flexibilité des brins n'étant obtenue que par leur seule forme.

Toujours dans un mode de réalisation préférée de l'invention, la lentille est livrée en place dans l'injecteur, ce qui dispense le chirurgien d'avoir à la charger et constitue un ensemble stérile prêt à l'emploi. Selon le mode de stérilisation utilisé, la lentille peut ou non être conditionnée à sec ou immergée dans un liquide, à l'intérieur du corps de seringue : dans ce deuxième cas, l'ensemble est équipé de joints d'étanchéité pour le piston, et d'un bouchon adapté à l'embout.

On a décrit ci-après, à titre d'exemples non limitatifs plusieurs modes de réalisation de l'injecteur selon l'invention, avec référence aux dessins annexés dans lesquels :
- la figure 1 est une vue en perspective du corps de l'injecteur,
- la figure 2 est une vue en perspective du piston de l'injecteur, avec la lentille non déformée prête à l'injection,
- la figure 3 est une vue en perspective montrant le piston monté dans le corps de seringue, avec la lentille non déformée prête à l'injection,
- les figures 4 A à 4 E sont des vues en coupe du corps suivant les plans A-A, B-B, C-C, D-D et E-E au moment où la lentille passe par ces plans,
- les figures 5 A à 5 F. sont des vues en coupe du corps suivant les plans A-A, B-B, C-C, D-D et E-E au moment ou les extrémités du piston passent par ces plans,
- les figures 6 A à 6 E sont des vues semblables aux figures 5 A à 5 E d'un second mode de réalisation,
- les figures 7 A à 7 E sont des vues semblables aux figures 5 A à 5 E d'un troisième mode de réalisation.
- les figures 8 A à 8 E sont des vues semblables aux figures 5 A à 5 E d'un quatrième mode de réalisation,
- les figures 9 A et 9 B sont des vues semblables aux figures 2 et 3, avec la lentille en cours d'injection, partiellement engagée dans l'embout,
- les figures 10 A et 10 B sont des vues semblables aux figures 2 et 3, avec la lentille en cours d'injection, partiellement libérée à l'extrémité de l'embout,
- les figures 11 A et 11 B montrent les mêmes éléments, au même stade que les figures 10 A et 10 B, avec l'injecteur retourné, présentant son biseau vers le bas.
- la figure 12 est une vue en perspective du piston seul dans son second mode de réalisation, non déformé, avant son montage dans le corps de seringue.

Tel qu'il est représenté aux dessins, l'injecteur de lentille selon l'invention comprend un corps de seringue monobloc et un piston qui sont désignés aux dessins d'une façon générale respectivement par les références 1 et 2. Le corps 1 comprend une partie 3 dont la section est semi-circulaire, avec une face bombée 3a et une face plane 3b, sa largeur interne maximale étant sensiblement égale à celle d'une lentille intra-oculaire 4 à plat (figure 4 A). Cette partie 3 est suivie d'une partie conique 5 qui se raccorde progressivement à une partie pratiquement cylindrique 6. La partie 5 présente une face bombée 5a et une face plane trapézoïdale 5b. Les diamètres internes de la partie 6 sont tels que la lentille 4 repliée sur clic même puisse s'y loger, soit environ 1,6 x 2,3 mm (figure 4 E). La partie 6 se termine par un embout d'injection 7 dont l'extrémité peut être droite ou biseautée et dont les diamètres extérieurs sont d'environ 1,9 x 2,6 mm. Suivant le sens d'ouverture préféré pour la lentille, l'éventuel biseau peut être orienté du côté de la face bombée, comme sur les dessins, ou du côté opposé.

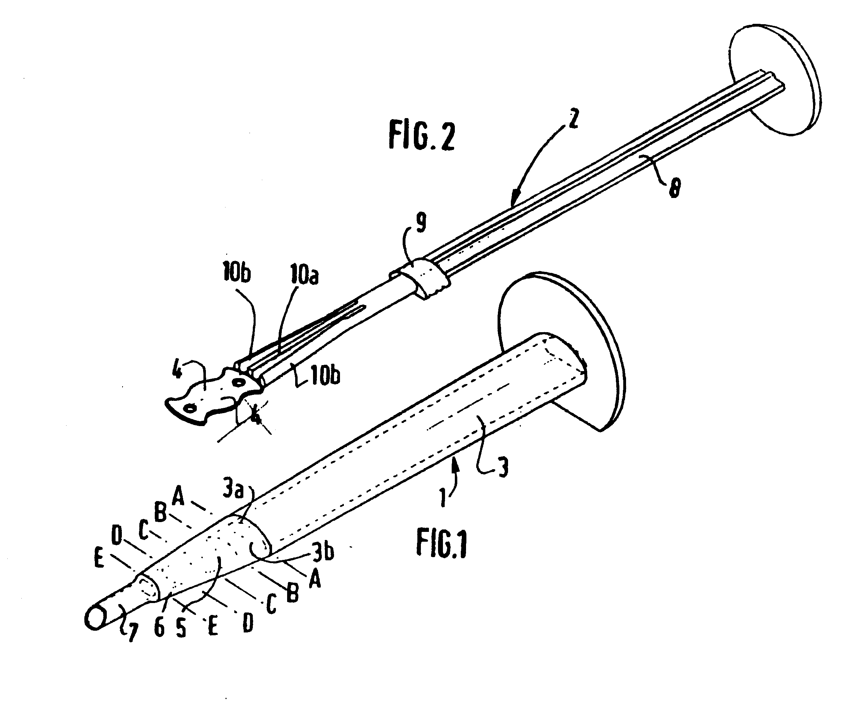

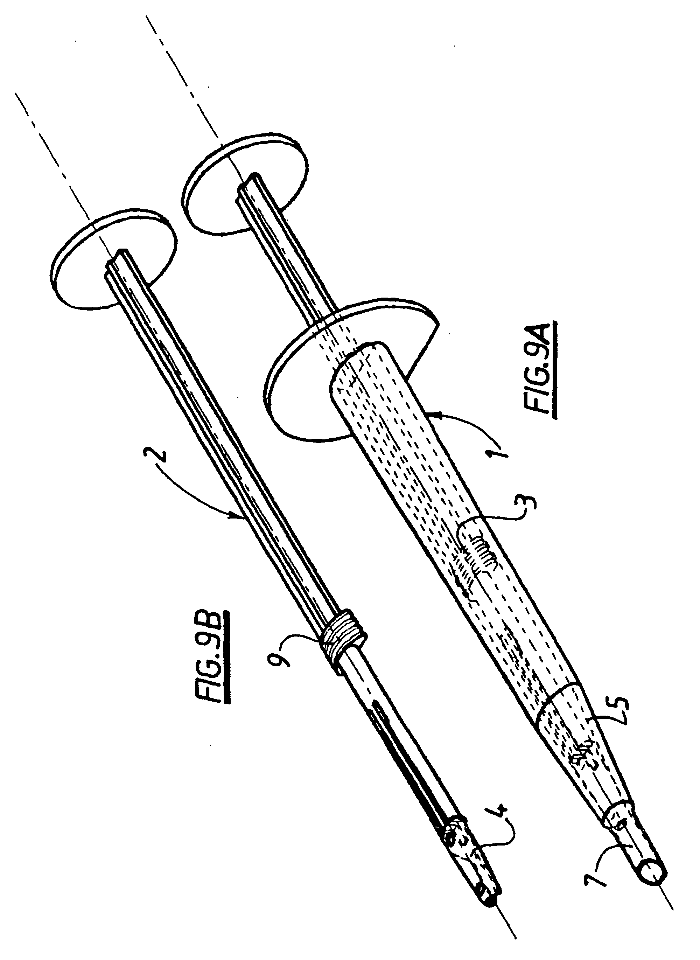

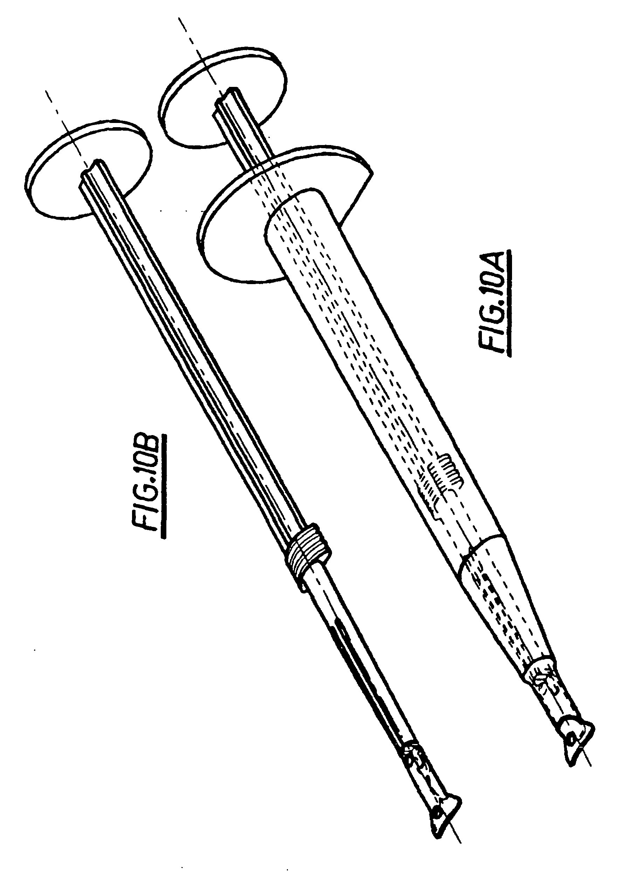

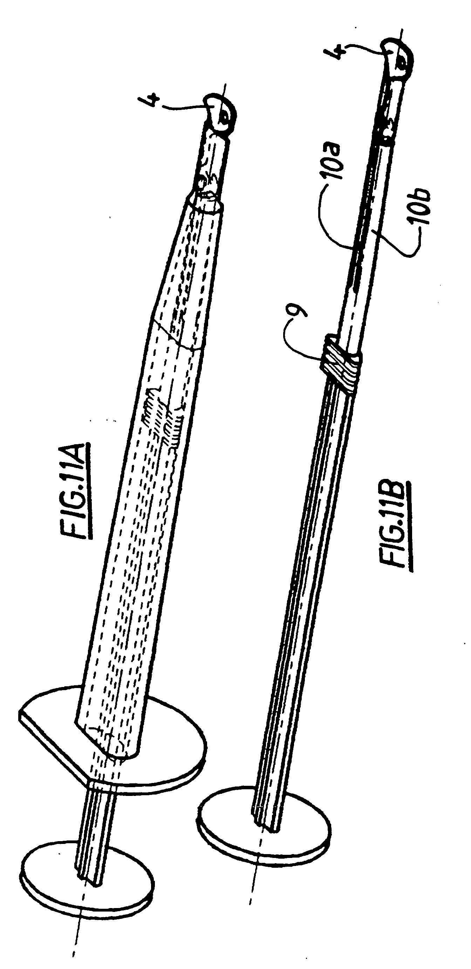

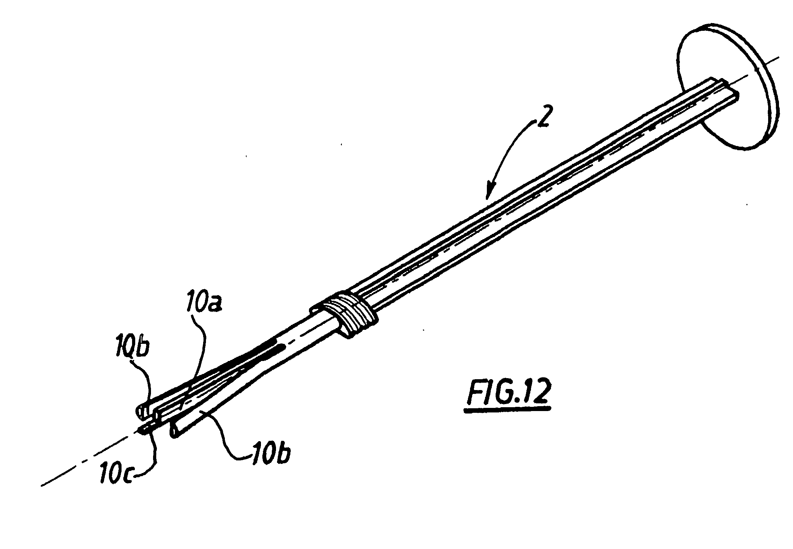

Dans le mode de réalisation des figures 1 à 3 et 9 à 12, le piston 2 comprend une partie cruciforme 8 terminée par une tête de guidage cylindrique 9 qui peut comporter des joints d'étanchéité et dont le diamètre est tel qu'elle puisse circuler librement dans la partie 3 du corps 1 en assurant le guidage du piston. Le piston comporte au-delà de la tête 9, une zone multibrins qui comprend, dans l'exemple représenté, un brin central 10a et deux brms latéraux 10b. Le brin central 10a est prolongé par une spatule 10c interdisant à la lentille de se déformer en direction de la face plane 3b du corps.

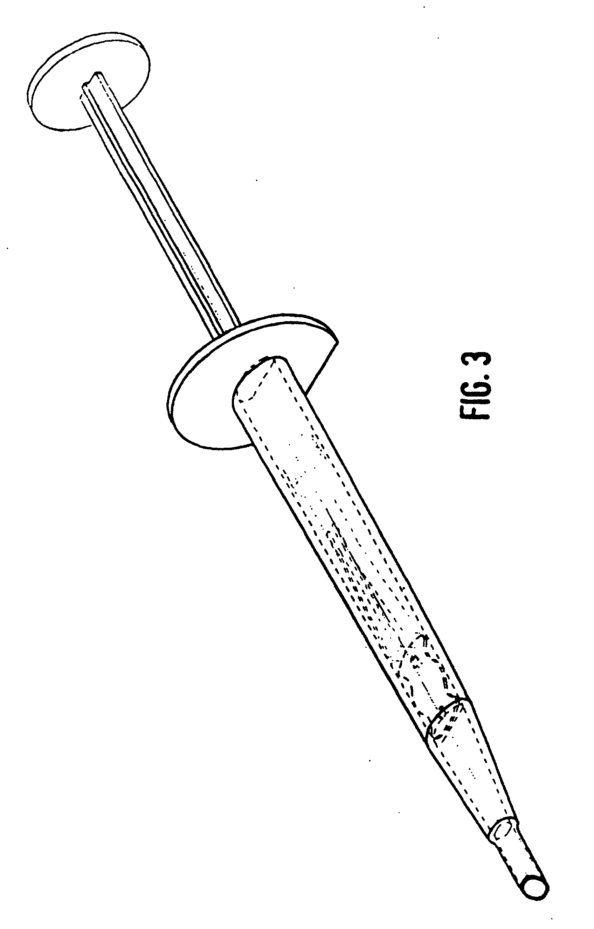

Pour utiliser cet injecteur, la lentille est placée dans la partie 3 du corps 1 et le piston est monté dans ce corps jusqu'à atteindre la position représentée par les figures 3, 4 A et 5 A. L'ensemble stérilisé ou monté aseptiquement parvient entre les mains du chirurgien, qui retire l'éventuel bouchon, et dispose dans la partie conique 5 du corps 1, selon sa technique personnelle, l'éventuelle solution visco-élastique lubrifiante, destinée à améliorer l'injection de la lentille.

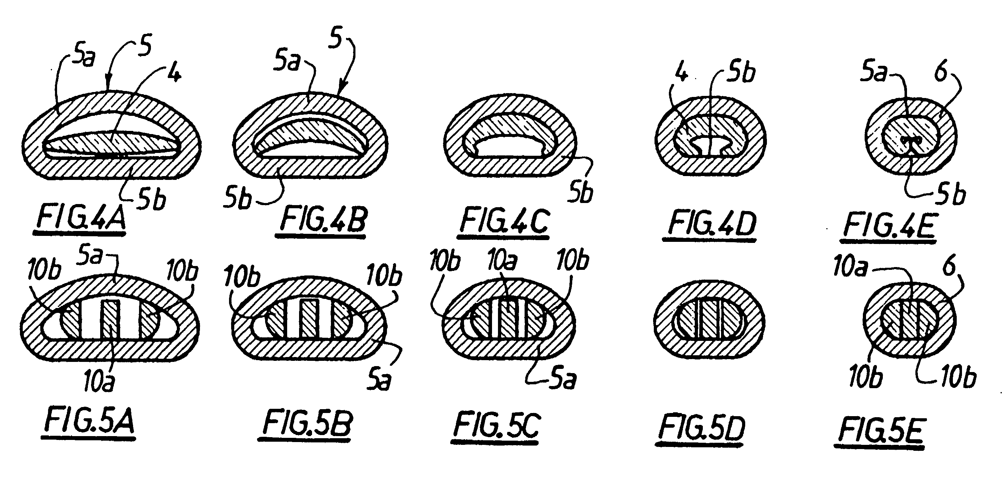

En partant de l'injecteur ainsi préparé, le chirurgien pousse sur le piston 2 et la lentille 4 est déplacée dans la zone conique 5 du corps : la lentille se trouve comprimée entre deux points diamétralement opposés, ce qui provoque son flambage en direction de la face bombée 5a du corps 1 (figure 4 B) car l'autre face 3b - 5b qui est plane et au départ plaquée contre l'optique (figure 4 A) interdit le flambage dans la direction opposée. Ensuite, la lentille vient au contact avec la face bombée 5a puis les bords libres plus minces commencent à se replier vers la face plane 5b (figure 4C). En même temps, les brins latéraux 10b se rapprochent l'un de l'autre (figures 4 C puis 5 C). Sous l'effet du rétrécissement de la section de la partie 5, les bords libres de la lentille 4 glissent contre la face plane 5b (figure 4 D). La partie centrale de la lentille 4 reste constamment plaquée contre la face bombée 5a et est ainsi stabilisée durant la poussée.

Une fois franchie la partie conique 5 du corps 1, les brins 10a et 10b se réunissent pour constituer un cylindre occupant pratiquement toute la section de l'extrémité 6 du corps 1 (figure 5 E). De son côté, la lentille 4 est roulée sur elle-même en occupant également toute cette section (figure 4 E). Lorsque la lentille est sur le point de déboucher, le chirurgien insère l'extrémité 7 dans l'incision, en orientant le biseau vers le bas. Puis en continuant la poussée sur le piston 2, il injecte progressivement la lentille à l'intérieur de l'oeil du patient, en l'engageant dans le sac capsulaire. En raison de son élasticité, la lentille se déploie pour reprendre sa forme initiale.

En fin de poussée, les trois brins dépassent légèrement l'extrémité du corps 1, de sorte que la lentille se trouve totalement libérée.

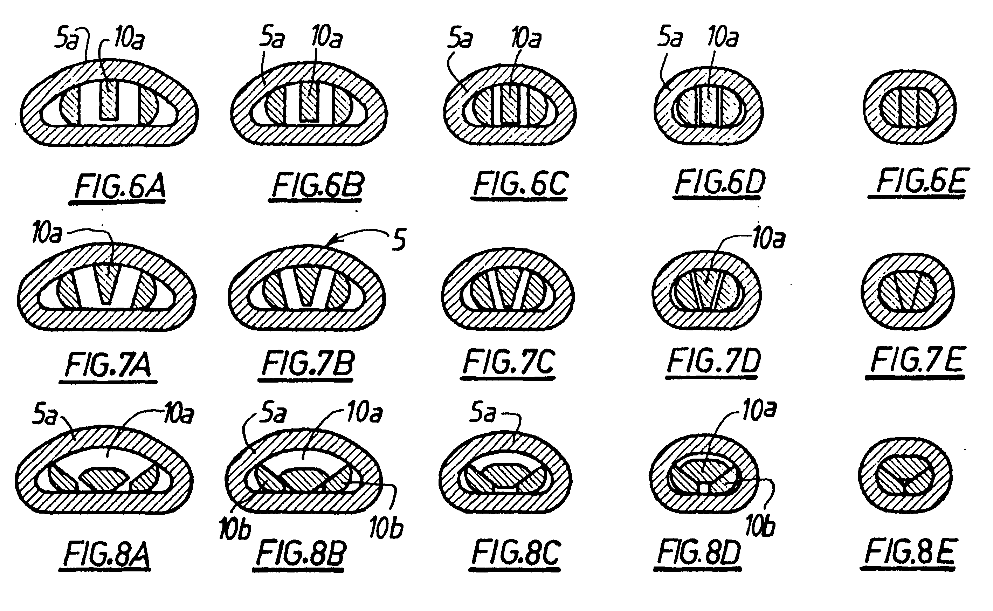

Le mode de réalisation des figures 6 A à 6 E est semblable à celui qui vient d'être décrit : il n'en diffère que par le fait que le brin central 10a est constamment en appui contre les parties bombées 3a et 5a du corps de l'injecteur.

Dans le mode de réalisation des figures 7 A à 7 E, similaire à la réalisation précédente, les plans de séparation entre le brin 10a et les brins 10b, au lieu d'être perpendiculaires à la face plane du corps, sont inclinés.

Dans le mode de réalisation des figures 8 A à 8 E, le brin central 10a est en forme de coin. En se rapprochant l'un de l'autre dans la partie conique 5, les brins latéraux 10b repoussent, par effet de coin, le brin central 10a en direction de la face bombée 5a, accompagnant ainsi le mouvement de la lentille.

Il va de soi que la présente invention ne doit pas être considérée comme limitée au mode de réalisation décrit et représenté, mais couvre, au contraire, toutes les variantes.

## Revendications

1. Dispositif pour l'infection d'une lentille intra-oculaire qui comprend un corps de seringue (1) dans lequel est monté un piston (2), l'ensemble pouvant être manipulé d'une seule main,
**caractérisé en ce que** le corps (1) est monobloc et comporte une partie cylindrique (3) pouvant contenir la lentille non déformée (4), un embout d'injection (6) ainsi qu'une partie intermédiaire conique (5), et ne présente ni ouverture, ni système annexe en forme de cartouche, volet, tiroir ou embout amovible, pour le chargement de cette lentille.

2. Dispositif selon la revendication 1,
**caractérisé en ce que** le corps de seringue (1) présente une face longitudinale interne pratiquement plane, la partie cylindrique (3) et la partie intermédiaire conique (5) ayant des sections approximativement semi-circulaires.

3. Dispositif selon la revendication 1 ou 2,
**caractérisé en ce que** l'extrémité d'éjection du piston comporte plusieurs brins (10a-10b), en matière plastique dure pouvant par flexibilité se rapprocher les uns des autres, lors du déplacement du piston en formant un cylindre occupant pratiquement toute la section de l'extrémité du corps (1), et pousser simultanément la lentille.

4. Dispositif selon la revendication 3,
**caractérisé en ce que** le brin central (10a) est en appui constant sur la paroi interne bombée du corps de seringue, pour limiter le risque de pincement de la lentille.

5. Dispositif selon la revendication 3,
**caractérisé en ce que** le brin central (10a) est en forme de coin, et qu'il est repoussé en direction de la paroi bombée du corps de seringue, sous l'effet du rapprochement des brins latéraux (10b).

6. Dispositif selon l'une des revendications 3 à 5,
**caractérisé en ce que** le brin unique ou le brin central (10a) est prolongé par une spatule (10c) maintenant la lentille du côté de la face bombée du corps, dans l'espace de poussée.

7. Dispositif selon l'une des revendications 1 à 6,
**caractérisé par** l'utilisation de joints d'étanchéité au niveau de la tête de guidage (9) et d'un bouchon obstruant l'extrémité (7), de manière à pouvoir conditionner directement la lentille en immersion dans un liquide.

8. Dispositif selon la revendication 7,
**caractérisé par** l'utilisation de matériaux résistant à la chaleur, pour permettre la stérilisation de l'ensemble (dispositif plus lentille), à l'autoclave.

## Patentansprüche

1. Vorrichtung zum Einsetzen einer interokularen Linse, umfassend ein Spritzengehäuse (1), in welchem ein Kolben (2) angeordnet ist, wobei die Anordnung mit einer einzigen Hand gehandhabt werden kann, **dadurch gekennzeichnet, dass** das Gehäuse (1) einstückig ist und einen zylindrischen Abschnitt (3) hat, welcher die nicht verformte Linse (4) aufnehmen kann, ferner einen Einführansatz (6) sowie einen konischen Zwischenabschnitt (5), und dass es weder eine Öffnung noch ein Anbausystem in Form einer Kartusche, einer Klappe, eines Schiebers oder abnehmbaren Ansatzstückes für das Laden dieser Linse aufweist.

2. Vorrichtung nach Anspruch 1, **dadurch gekennzeichnet, dass** das Spritzengehäuse (1) eine im wesentlichen ebene innere Längsfläche aufweist, wobei der zylindrische Abschnitt (3) und der konische Zwischenabschnitt (5) im wesentlichen halbkreisförmige Querschnitte haben.

3. Vorrichtung nach Anspruch 1 oder 2, **dadurch gekennzeichnet, dass** das Ausstoßende des Kolbens mehrere Stränge (10a - 10b) aus einem harten Plastikmaterial aufweist, die sich infolge ihrer Flexibilität einander annähern können, wobei sie bei der Verschiebung des Kolbens einen Zylinder bilden, welcher im wesentlichen den gesamten Querschnitt des Endes des Gehäuses (1) einnimmt, und die gleichzeitig die Linse weiterschieben können.

4. Vorrichtung nach Anspruch 3, **dadurch gekennzeichnet, dass** der zentrale Strang (10a) ständig an der gewölbten inneren Wand des Gehäuses der Spritze anliegt, um das Risiko eines Quetschens der Linse zu begrenzen.

5. Vorrichtung nach Anspruch 3, **dadurch gekennzeichnet, dass** der zentrale Strang (10a) die Form eines Keils hat, und dass er unter der Wirkung der Annäherung der seitlichen Einzelstäbe (10b) in Richtung der gewölbten Wand des Gehäuses der Spritze gedrückt wird.

6. Vorrichtung nach einem der Ansprüche 3 bis 5, **dadurch gekennzeichnet, dass** der einzige Strang oder der zentrale Strang (10a) durch einen Spatelabschnitt (10c) verlängert ist, welcher im Verschiebebereich die Linse auf Seite der gewölbten Fläche hält.

7. Vorrichtung nach einem der Ansprüche 1 bis 6, **gekennzeichnet durch** die Verwendung von Dichtungen im Bereich des Führungskopfes (9) und eines das Ende (7) verschließenden Stöpsels derart, dass man die Linse direkt in eine Flüssigkeit getaucht konditionieren kann.

8. Vorrichtung nach Anspruch 7, **gekennzeichnet durch** die Verwendung eines gegen Wärme beständigen Materials, um die Sterilisierung der Gesamtanordnung (Vorrichtung plus Linse) im Autoklaven zu ermöglichen.

## Claims

1. A device for injecting an intraocular lens, the device comprising a syringe body (1) in which a piston (2) is mounted, the assembly being suitable for handling in one hand, the device being **characterized in that** the body (1) is a single piece and comprises a cylindrical portion (3) capable of containing the lens (4) when not deformed, an injection endpiece (6), and a conical intermediate portion (5), and presents no opening, no auxiliary system, in the form of a cartridge, flap, slide, or removable endpiece, for loading the lens.

2. A device according to claim 1, **characterized in that** the syringe body (1) has an internal longitudinal face that is practically plane, the cylindrical portion (3) and the conical intermediate portion (5) having sections that are approximately semicircular.

3. A device according to claim 1 or claim 2, **characterized in that** the injection end of the piston comprises a plurality of fingers (10a-10b) of hard plastic material capable of flexing towards one another as the piston moves so as to form a cylinder that occupies practically the entire section of the end of the body (1), while simultaneously pushing the lens.

4. A device according to claim 3, **characterized in that** the central finger (10a) bears constantly against the curved inside wall of the syringe body so as to limit the risk of the lens becoming jammed.

5. A device according to claim 3, **characterized in that** the central finger (10a) is wedge-shaped and is urged towards the curved wall of the syringe body under the effect of the side fingers (10b) moving towards each other.

6. A device according to any one of claims 3 to 5, **characterized in that** the single finger or central finger (10a) is extended by a spatula (10c) holding the lens beside the curved face of the body in the thrust space.

7. A device according to any one of claims 1 to 6, **characterized by** using sealing gaskets at the guide head (9) and a stopper closing the end (7) so as to make it possible for the lens to be packaged directly in immersion in a liquid.

8. A device according to claim 7, **characterized by** the use of materials that withstand heat, to enable the assembly (device plus lens) to be sterilized in an autoclave.
